# EUROPEAN PATENT SPECIFICATION

(11) **EP 0 897 006 B1**
(45) Date of publication and mention of the grant of the patent: **01.12.2004**
(21) Application number: 97305968.6
(22) Date of filing: 06.08.1997
(51) Int. Cl.: C12N 15/53, C12N 1/21, C12N 9/06

(54) **Rhodosporidium d-amino acid oxidase**
D-Aminosäure Oxidase aus Rhodosporidium
Oxydase de D-acide aminé de Rhodosporidium

(43) Date of publication of application: 17.02.1999
(73) Proprietor: Food Industry Research and Development Institute, Hsinchu 30099 (TW)
(72) Inventor: Liao, Gwo-Jen, Hsinchu 30099 (TW); Yi-Jang, Lee, Hsinchu 30099 (TW); Yun-Huey, Lee, Kaohsiung (TW); Li-Lin, Chen, Hsinchu 30099 (TW); Wen-Shen, Chu, Hsinchu 30099 (TW)
(74) Representative: Hall, Robert Leonard

(56) References cited:
- EP-A- 0 364 275
- EP-A- 0 496 993
- WO-A-96/27667
- IN-WOOK KIM ET AL: "SIMPLE AND RAPID DETERMINATION OF THE ACTIVITY OF RECOMBINANT D-AMINO ACID OXIDASE IN CEPHALOSPORIN C BIOCONVERSION WITH USE OF A MICRO PO2 PROBE" BIOTECHNOLOGY TECHNIQUES, vol. 9, no. 12, December 1995, pages 863-868, XP000677601
- FAOTTO, LUDOVICA ET AL: "Amino acid sequence of D-amino acid oxidase from the yeast Rhodotorula gracilis" FLAVINS FLAVOPROTEINS, PROCEEDINGS OF THE INTERNATIONAL SYMPOSIUM,11TH (1994), MEETING DATE 1993, EDITOR(S): YAGI, KUNIO. PUBLISHER: DE GRUYTER, BERLIN, GERMANY, 1993, pages 163-166, XP000677151
- FAOTTO L ET AL: "THE PRIMARY STRUCTURE OF D-AMINO ACID OXIDASE FROM RHODOTORULA GRACILIS" BIOTECHNOLOGY LETTERS, vol. 17, no. 2, 1 February 1995, pages 193-198, XP000574023
- SIMONETTA M P ET AL: "PROPERTIES OF D-AMINO-ACID OXIDASE FROM RHODOTORULA GRACILIS" EUROPEAN JOURNAL OF BIOCHEMISTRY, vol. 180, no. 1, March 1989, pages 199-204, XP000677165
- W.H. LEE ET AL: "D-Amino acid oxidase activity from Rhodosporidium toruloides" ABSTRACTS OF THE ANNUAL MEETING OF THE AMERICAN SOCIETY FOR MICROBIOLOGY, vol. 95, no. 0, May 1995, WASHINGTON US, page 368 XP002050710
- FUKUI K ET AL: "MOLECULAR CLONING AND SEQUENCES ANALYSIS OF CDNAS ENCODING PORCINE KIDNEY D-AMINO ACID OXIDASE" BIOCHEMISTRY, vol. 26, no. 12, 16 June 1987, pages 3612-3618, XP002034043

### SEQUENCE LISTING

(1) GENERAL INFORMATION
   (i) APPLICANT: Food Industry Research and Development Institute
   (ii) TITLE OF THE INVENTION: RHODOSPORIDIUM D-AMINO ACID OXIDASE
   (iii) NUMBER OF SEQUENCES: 18
   (iv) CORRESPONDENCE ADDRESS:
      (A) ADDRESSEE: Dibb Lupton Alsop
      (B) STREET: Fountain Precinct, Balm Green
      (C) CITY: Sheffield
      (D) POST CODE: S1 1RZ
      (E) COUNTRY: UK
   (v) COMPUTER READABLE FORM:
      (A) MEDIUM TYPE: Diskette
      (B) COMPUTER: IBM Compatible
      (C) OPERATING SYSTEM: Windows95
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER:
      (B) FILING DATE:
   (viii) ATTORNEY/AGENT INFORMATION:
      (A) NAME: HALL, Robert Leonard
   (ix) TELECOMMUNICATION INFORMATION:
      (A) TELEPHONE: +44 114 283 3253
      (B) TELEFAX: +44 114 273 0312
(2) INFORMATION FOR SEQ ID NO:1:
   (i) SEQUENCE CHARACTERISTICS:
      (A) LENGTH: 1458 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:1:
   ATGCACTCTC AGAAGCGCGT CGTTGTCCTC GGATCAGGCG GTGCGTCTTT TCCCTCTCCT      60
   CCCCACACCC GACAGTCCTC GACGAGGTGT AGGACGGCGA GCAAAGCTGC CGAGGGCGAT     120
   CTGGGCTGAC TGAGCGCTCG AGTGTACAGT TATCGGTCTG AGCAGCGCCC TCATCCTCGC     180
   TCGGAAGGGC TACAGCGTGC ATATTCTCGC GCGCGACTTG CCGGAGGACG TCTCGAGCCA     240
   GACTTTCGCT TCACCATGGG CTGTGCGTCG TCTCACTGTA GTTGGAGGAT GTCAGCGAGA     300
   GCTGATCAAT CTCGTCATCC CCGCAGGGCG CGAATTGGAC GCCTTTCATG ACGCTTACAG     360
   ACGGTCCTCG ACAAGCAAAA TGGGAAGAAT CGACTTTGTG CGTCTCCTTC TACCTCATTC     420
   TTGGCCTCGA GCTGACGAGT GTATGATACA CAGCAAGAAG TGGGTCGAGT TGGTCCCGAC     480
   GGGCCATGCC ATGTGGCTCA AGGGGACGAG GCGGTTCGCG CAGAACGAAG ACGGCTTGCT     540
   CGGGCACTGG TACAAGGACA TCACGCCAAA TGTGCGCCCA CATTCACTCT TCCCTTCGCA     600
   TGTCTCCGTT TACTGACCCG CCCTCTTTCG CCGTGCGCAG TACCGCCCCC TCCCATCTTC     660
   CGAATGTCCA CCTGGCGCTA TCGGCGTAAC CTACGACACC CTCTCCGTCC ACGCACCAAA     720
   GTACTGCCAG TACCTTGCAA GAGAGCTGCA GAAGCTCGGC GCGACGTTTG AGAGACGGAC     780
   CGTTACGTCG CTTGAGCAGG CGTTCGACGG TGCGGATTTG GTGGTCAACG CTACGGGACT     840
   TGGTATGTCC CGAACTGCCC CTCTCTACCT GCAATTTTGC TGATTGATAT GCTCGCAGGC     900
   GCCAAGTCGA TTGCGGGCAT CGACGACCAA GCCGCCGAGC CAATCCGCGG CCAAACCGTC     960
   CTCGTCAAGT CCCCATGCAA GCGATGCACG ATGGACTCGT CCGACCCCGC TTCTCCCGCC    1020
   TACATCATTC CCCGACCAGG TGGCGAAGTC ATCTGCGGCG GGACGTACGG CGTGGGAGAC    1080
   TGGGACTTGT CTGTCAACCC AGAGACGGTC CAGCGGATCC TCAAGCACTG CTTGCGCCTC    1140
   GACCCGACCA TCTCGAGCGA CGGAACGATC GAAGGCATCG AGGTCCTCCG CCACAACGTC    1200
   GGCTTGCGAC CTGCACGACG AGGCGGACCC CGCGTCGAGG CAGAACGGAT CGTCCTGCCT    1260
   CTCGACCGGA CAAAGTCGCC CCTCTCGCTC GGCAGGGGCA GCGCACGAGC CGCGAAGGAG    1320
   AAGGAGGTCA CGCTTGTGCA TGCGTATGGC TTCTCGAGTG CGGGATACCA GCAGAGTTGG    1380
   GGCGCGGCGG AGGATGTCGC GCAGCTCGTC GACGAGGCGT TCCAGCGGTA CCACGGCGCG    1440
   GCGCGGGAGT CGAAGTTG                                                  1458
(2) INFORMATION FOR SEQ ID NO:2:
   (i) SEQUENCE CHARACTERISTICS:
      (A) LENGTH: 1104 base pairs
      (B) TYPE: nucleic acid
      (C) STRANDEDNESS: single
      (D) TOPOLOGY: linear
   (ii) MOLECULE TYPE: cDNA
   (ix) FEATURE:
      (A) NAME/KEY: Coding Sequence
      (B) LOCATION: 1...1104
   (xi) SEQUENCE DESCRIPTION: SEQ ID NO:2:
   ATG CAC TCT CAG AAG CGC GTC GTT GTC CTC GGA TCA GGC GTT ATC GGT      48
   Met His Ser Gln Lys Arg Val Val Val Leu Gly Ser Gly Val Ile Gly
     1               5                  10                  15
   CTG AGC AGC GCC CTC ATC CTC GCT CGG AAG GGC TAC AGC GTG CAT ATT      96
   Leu Ser Ser Ala Leu Ile Leu Ala Arg Lys Gly Tyr Ser Val His Ile
                20                  25                  30
   CTC GCG CGC GAC TTG CCG GAG GAC GTC TCG AGC CAG ACT TTC GCT TCA     144
   Leu Ala Arg Asp Leu Pro Glu Asp Val Ser Ser Gln Thr Phe Ala Ser
            35                  40                  45
   CCA TGG GCT GGC GCG AAT TGG ACG CCT TTC ATG ACG CTT ACA GAC GGT     192
   Pro Trp Ala Gly Ala Asn Trp Thr Pro Phe Met Thr Leu Thr Asp Gly
        50                   55                   60
   CCT CGA CAA GCA AAA TGG GAA GAA TCG ACT TTC AAG AAG TGG GTC GAG      240
   Pro Arg Gln Ala Lys Trp Glu Glu Ser Thr Phe Lys Lys Trp Val Glu
    65                  70                   75                  80
   TTG GTC CCG ACG GGC CAT GCC ATG TGG CTC AAG GGG ACG AGG CGG TTC      288
   Leu Val Pro Thr Gly His Ala Met Trp Leu Lys Gly Thr Arg Arg Phe
                       85                   90                   95
   GCG CAG AAC GAA GAC GGC TTG CTC GGG CAC TGG TAC AAG GAC ATC ACG      336
   Ala Gln Asn Glu Asp Gly Leu Leu Gly His Trp Tyr Lys Asp Ile Thr
               100                  105                  110
   CCA AAT TAC CGC CCC CTC CCA TCT TCC GAA TGT CCA CCT GGC GCT ATC      384
   Pro Asn Tyr Arg Pro Leu Pro Ser Ser Glu Cys Pro Pro Gly Ala Ile
           115                  120                  125
   GGC GTA ACC TAC GAC ACC CTC TCC GTC CAC GCA CCA AAG TAC TGC CAG      432
   Gly Val Thr Tyr Asp Thr Leu Ser Val His Ala Pro Lys Tyr Cys Gln
       130                  135                  140
   TAC CTT GCA AGA GAG CTG CAG AAG CTC GGC GCG ACG TTT GAG AGA CGG      480
   Tyr Leu Ala Arg Glu Leu Gln Lys Leu Gly Ala Thr Phe Glu Arg Arg
   145                  150                  155                 160
   ACC GTT ACG TCG CTT GAG CAG GCG TTC GAC GGT GCG GAT TTG GTG GTC      528
   Thr Val Thr Ser Leu Glu Gln Ala Phe Asp Gly Ala Asp Leu Val Val
                   165                  170                  175
   AAC GCT ACG GGA CTT GGC GCC AAG TCG ATT GCG GGC ATC GAC GAC CAA      576
   Asn Ala Thr Gly Leu Gly Ala Lys Ser Ile Ala Gly Ile Asp Asp Gln
               180                  185                  190
   GCC GCC GAG CCA ATC CGC GGC CAA ACC GTC CTC GTC AAG TCC CCA TGC      624
   Ala Ala Glu Pro Ile Arg Gly Gln Thr Val Leu Val Lys Ser Pro Cys
           195                  200                  205
   AAG CGA TGC ACG ATG GAC TCG TCC GAC CCC GCT TCT CCC GCC TAC ATC      672
   Lys Arg Cys Thr Met Asp Ser Ser Asp Pro Ala Ser Pro Ala Tyr Ile
       210                 215                 220
   ATT CCC CGA CCA GGT GGC GAA GTC ATC TGC GGC GGG ACG TAC GGC GTG     720
   Ile Pro Arg Pro Gly Gly Glu Val Ile Cys Gly Gly Thr Tyr Gly Val
   225                 230                 235                 240
   GGA GAC TGG GAC TTG TCT GTC AAC CCA GAG ACG GTC CAG CGG ATC CTC     768
   Gly Asp Trp Asp Leu Ser Val Asn Pro Glu Thr Val Gln Arg Ile Leu
                   245                 250                 255
   AAG CAC TGC TTG CGC CTC GAC CCG ACC ATC TCG AGC GAC GGA ACG ATC     816
   Lys His Cys Leu Arg Leu Asp Pro Thr Ile Ser Ser Asp Gly Thr Ile
               260                 265                 270
   GAA GGC ATC GAG GTC CTC CGC CAC AAC GTC GGC TTG CGA CCT GCA CGA     864
   Glu Gly Ile Glu Val Leu Arg His Asn Val Gly Leu Arg Pro Ala Arg
           275                 280                 285
   CGA GGC GGA CCC CGC GTC GAG GCA GAA CGG ATC GTC CTG CCT CTC GAC     912
   Arg Gly Gly Pro Arg Val Glu Ala Glu Arg Ile Val Leu Pro Leu Asp
       290                 295                 300
   CGG ACA AAG TCG CCC CTC TCG CTC GGC AGG GGC AGC GCA CGA GCC GCG     960
   Arg Thr Lys Ser Pro Leu Ser Leu Gly Arg Gly Ser Ala Arg Ala Ala
   305                 310                 315                 320
   AAG GAG AAG GAG GTC ACG CTT GTG CAT GCG TAT GGC TTC TCG AGT GCG    1008
   Lys Glu Lys Glu Val Thr Leu Val His Ala Tyr Gly Phe Ser Ser Ala
                   325                 330                 335
   GGA TAC CAG CAG AGT TGG GGC GCG GCG GAG GAT GTC GCG CAG CTC GTC    1056
   Gly Tyr Gln Gln Ser Trp Gly Ala Ala Glu Asp Val Ala Gln Leu Val
               340                 345                 350
   GAC GAG GCG TTC CAG CGG TAC CAC GGC GCG GCG CGG GAG TCG AAG TTG
   1104
   Asp Glu Ala Phe Gln Arg Tyr His Gly Ala Ala Arg Glu Ser Lys Leu
           355                 360                 365
(2) INFORMATION FOR SEQ ID NO:3:
   (i) SEQUENCE CHARACTERISTICS:
      (A) LENGTH: 368 amino acids
      (B) TYPE: amino acid
      (D) TOPOLOGY: linear
   (ii) MOLECULE TYPE: protein
   (v) FRAGMENT TYPE: internal
   (xi) SEQUENCE DESCRIPTION: SEQ ID NO:3:
   Met His Ser Gln Lys Arg Val Val Val Leu Gly Ser Gly Val Ile Gly
     1               5                  10                  15
   Leu Ser Ser Ala Leu Ile Leu Ala Arg Lys Gly Tyr Ser Val His Ile
               20                  25                  30
   Leu Ala Arg Asp Leu Pro Glu Asp Val Ser Ser Gln Thr Phe Ala Ser
           35                  40                  45
   Pro Trp Ala Gly Ala Asn Trp Thr Pro Phe Met Thr Leu Thr Asp Gly
       50                  55                  60
   Pro Arg Gln Ala Lys Trp Glu Glu Ser Thr Phe Lys Lys Trp Val Glu
    65                  70                  75                  80
   Leu Val Pro Thr Gly His Ala Met Trp Leu Lys Gly Thr Arg Arg Phe
                    85                  90                  95
   Ala Gln Asn Glu Asp Gly Leu Leu Gly His Trp Tyr Lys Asp Ile Thr
               100                 105                 110
   Pro Asn Tyr Arg Pro Leu Pro Ser Ser Glu Cys Pro Pro Gly Ala Ile
           115                 120                 125
   Gly Val Thr Tyr Asp Thr Leu Ser Val His Ala Pro Lys Tyr Cys Gln
       130                 135                 140
   Tyr Leu Ala Arg Glu Leu Gln Lys Leu Gly Ala Thr Phe Glu Arg Arg
   145                 150                 155                 160
   Thr Val Thr Ser Leu Glu Gln Ala Phe Asp Gly Ala Asp Leu Val Val
                   165                 170                 175
   Asn Ala Thr Gly Leu Gly Ala Lys Ser Ile Ala Gly Ile Asp Asp Gln
               180                 185                 190
   Ala Ala Glu Pro Ile Arg Gly Gln Thr Val Leu Val Lys Ser Pro Cys
               195                 200                 205
   Lys Arg Cys Thr Met Asp Ser Ser Asp Pro Ala Ser Pro Ala Tyr Ile
       210                 215                 220
   Ile Pro Arg Pro Gly Gly Glu Val Ile Cys Gly Gly Thr Tyr Gly Val
   225                 230                 235                 240
   Gly Asp Trp Asp Leu Ser Val Asn Pro Glu Thr Val Gln Arg Ile Leu
                   245                 250                 255
   Lys His Cys Leu Arg Leu Asp Pro Thr Ile Ser Ser Asp Gly Thr Ile
               260                 265                 270
   Glu Gly Ile Glu Val Leu Arg His Asn Val Gly Leu Arg Pro Ala Arg
           275                 280                 285
   Arg Gly Gly Pro Arg Val Glu Ala Glu Arg Ile Val Leu Pro Leu Asp
       290                 295                 300
   Arg Thr Lys Ser Pro Leu Ser Leu Gly Arg Gly Ser Ala Arg Ala Ala
   305                 310                 315                 320
   Lys Glu Lys Glu Val Thr Leu Val His Ala Tyr Gly Phe Ser Ser Ala
                   325                 330                 335
   Gly Tyr Gln Gln Ser Trp Gly Ala Ala Glu Asp Val Ala Gln Leu Val
               340                 345                 350
   Asp Glu Ala Phe Gln Arg Tyr His Gly Ala Ala Arg Glu Ser Lys Leu
           355                 360                 365
(2) INFORMATION FOR SEQ ID NO:4:
   (i) SEQUENCE CHARACTERISTICS:
      (A) LENGTH: 190 base pairs
      (B) TYPE: nucleic acid
      (C) STRANDEDNESS: double
      (D) TOPOLOGY: linear
   (ii) MOLECULE TYPE: Genomic DNA
   (xi) SEQUENCE DESCRIPTION: SEQ ID NO:4:
   AAGCTTCGGC ACGAGCATGA GTGTGAATGA TGGTCCAAGG AGGACAGCGC AGAGTCAACA    60
   GGAGGGCACA TGGAGGCAGA GCGTGGGGCG GAGGAGGCAG ATGGGGAGTC GCGCTGGGGG   120
   ACGAGGGGGT GTCGCTCGAC TAACAGCTCT CTATCGCTCT TGCTGCTGCT TGTACTACTC   180
   GAACGACGCC                                                          190
(2) INFORMATION FOR SEQ ID NO:5
   (i) SEQUENCE CHARACTERISTICS:
      (A) LENGTH: 801 base pairs
      (B) TYPE: nucleic acid
      (C) STRANDEDNESS: single
      (D) TOPOLOGY: linear
   (ii) MOLECULE TYPE: Genomic DNA
   (xi) SEQUENCE DESCRIPTION: SEQ ID NO:5:
   GGCGGGATTT GTGGCTGTAT TGCGGGCATC TACAAGACCA GCTTCATCTC GGACGACAAC     60
   ACGAGAGCGG CGAGTCTTCG TACCGTCTGA CCTTCGCAAT CCGCCGAGTC CTTGCCCGTT    120
   GCCCTGCTTG CTCCTTTCGT ATCTCCTGTG ACTCGGAACG TCGCTCTTCG CCTCTGTCAC    180
   TTGCCAGGCC GTCCCTTCAA ACTGTCGCCG CCCTCCTCCT CCCAATCTCC TCCTCACGCG    240
   ACTTGCCACT ACTCTCACTT CTCCTGCTTA CCAAGGCTTA CACTACGCAA CACTAAGCGC    300
   GGCATCAGGT TCCGTTGCGC GCCTCGTCAC GACCCGACTT TTTTTCGCCC GTTCGCTCGC    360
   CTCGCTCCGT TGCCGAGCGA AGAACTTCGC CTGCCTTCGA ATCTCTCGCC TTGCTCGTCT    420
   CGTCCTGCTC CGTTCCACCA CAGATAGACT CACAGCAACA CACTCACAAT GGTCAAGGTG    480
   CGCACCCACT CCCGCCAGTC CAACTCGCGC GACGAGAGAC CACCGTCTCG TTGACATCAC    540
   TGACCTCGTC GCTCGCCACC CACCCTCCCG CTCGTCCATG CACACAGGCA GCACCACGAT    600
   CAGACGAGCA GCAGCACTAC CACGCCACAG TCATGGGCGG ACTCAAGGGC GGCGCGATGG    660
   GGTTGGCGGC GGGTGGAGCG GGTGCGGTTG CGCTGCAGAG GGCGAACGTA CAGGCGTTCA    720
   CGAGGTTGAC GCTGCCTCTC AAGGCGTTTG CTGTCACGTA CGTCCCGCGA TCCCTTACTA    780
   TGCGACTCCC TCGGTGAATT C                                              801
(2) INFORMATION FOR SEQ ID NO:6:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: degenerate oligonucleotide
   (iv) SEQUENCE DESCRIPTION SEQ ID NO:6:
   AARTAYTGYC ARTAYC     16
(2) INFORMATION FOR SEQ ID NO:7:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: degenerate oligonucleotide
   (xi) SEQUENCE DESCRIPTION: SEQ ID NO:7:
   ATNGAYGAYC AYGCNGC     17
(2) INFORMATION FOR SEQ ID NO:8:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: degenerate oligonucleotide
   (xi) SEQUENCE DESCRIPTION: SEQ ID NO:8:
   GCNGCYTGRT CRTCNAT 17
(2) INFORMATION FOR SEQ ID NO:9:
   (i) SEQUENCE CHARACTERISTICS:
      (A) LENGTH: 17 base pairs
      (B) TYPE: nucleic acid
      (C) STRANDEDNESS: single
      (D) TOPOLOGY: linear
   (ii) MOLECULE TYPE: degenerate oligonucleotide
   (xi) SEQUENCE DESCRIPTION: SEQ ID NO:9:
   ATGGAYAGYA GYGAYCC 17
(2) INFORMATION FOR SEQ ID NO:10:
   (i) SEQUENCE CHARACTERISTICS:
      (A) LENGTH: 22 amino acids
      (B) TYPE: amino acid
      (D) TOPOLOGY: linear
   (ii) MOLECULE TYPE: peptide
   (xi) SEQUENCE DESCRIPTION: SEQ ID NO:10:
   Met His Ser Gln Lys Arg Val Val Val Leu Gly Ser Gly Val Ile Gly
    1               5                  10                  15
   Leu Ser Ser Ala Leu Ile
               20
(2) INFORMATION FOR SEQ ID NO:11:
   (i) SEQUENCE CHARACTERISTICS:
      (A) LENGTH: 10 amino acids
      (B) TYPE: amino acid
      (D) TOPOLOGY: linear
   (ii) MOLECULE TYPE: peptide
   (xi) SEQUENCE DESCRIPTION: SEQ ID NO:11:
   Tyr Cys Gln Tyr Leu Ala Arg Glu Leu Gln
    1               5                   10
(2) INFORMATION FOR SEQ ID NO: 12 :
   (i) SEQUENCE CHARACTERISTICS:
      (A) LENGTH: 14 amino acids
      (B) TYPE: amino acid
      (D) TOPOLOGY: linear
   (ii) MOLECULE TYPE: peptide
   (xi) SEQUENCE DESCRIPTION: SEQ ID NO:12:
   Ile Ala Gly Gly Ile Asp Asp Gln Ala Ala Glu Pro Ile Arg
    1               5                   10
(2) INFORMATION FOR SEQ ID NO:13:
   (i) SEQUENCE CHARACTERISTICS:
      (A) LENGTH: 9 amino acids
      (B) TYPE: amino acid
      (D) TOPOLOGY: linear
   (ii) MOLECULE TYPE: peptide
   (xi) SEQUENCE DESCRIPTION: SEQ ID NO:13:
   Arg Cys Thr Met Asp Ser Ser Asp Pro
    1               5
(2) INFORMATION FOR SEQ ID NO:14:
   (i) SEQUENCE CHARACTERISTICS:
      (A) LENGTH: 8 amino acids
      (B) TYPE: amino acid
      (D) TOPOLOGY: linear
   (ii) MOLECULE TYPE: peptide
   (xi) SEQUENCE DESCRIPTION: SEQ ID NO:14:
   Ile Ala Gly Ile Asp Asp Gln Ala
   1 5
(2) INFORMATION FOR SEQ ID NO:15:
   (i) SEQUENCE CHARACTERISTICS:
      (A) LENGTH: 214 base pairs
      (B) TYPE: nucleic acid
      (C) STRANDEDNESS: double
      (D) TOPOLOGY: linear
   (ii) MOLECULE TYPE: genomic DNA
   (xi) SEQUENCE DESCRIPTION: SEQ ID NO:15:
   AAGTACTGCC AGTACCTTGC AAGAGAGCTG CAGAAGCTCG GCGCGACGTT TGAGAGACGG    60
   ACCGTTACGT CGCTTGAGCA GGCGTTCGAC GGTGCGGATT TGGTGGTCAA CGCTACGGGA   120
   CTTGGTATGT CCCGAACTGC CCCTCTCTAC CTGCAATTTT GCTGATTGAT ATGCTCGCAG   180
   GCGCCAAGTC GATTGCGGGC ATCGACGACC AAGC                               214
(2) INFORMATION FOR SEQ ID NO:16:
   (i) SEQUENCE CHARACTERISTICS:
      (A) LENGTH: 10 base pairs
      (B) TYPE: nucleic acid
      (C) STRANDEDNESS: double
      (D) TOPOLOGY: linear
   (ii) MOLECULE TYPE: Kozak sequence
   (xi) SEQUENCE DESCRIPTION: SEQ ID NO:16:
   CCACCATGGC    10
(2) INFORMATION FOR SEQ ID NO:17:
   (i) SEQUENCE CHARACTERISTICS:
      (A) LENGTH: 8 base pairs
      (B) TYPE: nucleic acid
      (C) STRANDEDNESS: double
      (D) TOPOLOGY: linear
   (ii) MOLECULE TYPE: consensus
   (xi) SEQUENCE DESCRIPTION: SEQ ID NO:17:
   YGTGTTYY 8
(2) INFORMATION FOR SEQ ID NO:18:
   (i) SEQUENCE CHARACTERISTICS:
      (A) LENGTH: 8 base pairs
      (B) TYPE: nucleic acid
      (C) STRANDEDNESS: double
      (D) TOPOLOGY: linear
   (ii) MOLECULE TYPE: genomic DNA
   (xi) SEQUENCE DESCRIPTION: SEQ ID NO:18:
   TGTATTGC 8

## Description

### Background of the Invention

D-amino acid oxidase ("DAO") is a flavoprotein known to be able to convert cephalosporin C ("Ceph C") to α-ketoadipyl cephalosporanic acid ("α-ketoadipyl 7-ACA), hydrogen peroxide, and ammonia. α-ketoadipyl 7-ACA, upon reaction with hydrogen peroxide, can be further transformed into glutaryl-7-aminocephalosporanic acid ("GL-7-ACA"). Since GL-7-ACA is a starting material for the production of cephem antibiotics, DAO is of great industrial interest.

Besides the production of α-keto acids (Tosa et al., Agric Biol Chem [1974] 38, 1529-1536), DAO has been used in the resolution of racemic mixtures of amino acids (Tu et al., Separation Sci [1972] 7, 403-408) and analytical determination of amino acids (Guilbault et al., Anal Chim Acta [1971] 56, 285-290). DAO can be found in various microorganisms, insects and animals.

### Summary of the Invention

This invention relates to an isolated nucleic acid encoding a DAO of the genus *Rhodosporidium*, e.g., *R*. *diobovatum*, *R. sphaerocarpum*, *R. kratochvilovae*, *R. toruloides*, *R*. *lusitaniae*, *R*. *paludignenum, R*. *dacryoidum*, *R*. *babjevae*, and *R*. *fluviale*. The term "D-amino acid oxidase of the genus *Rhodosporidium*" refers to any natural or man-made variant of DAO of the genus *Rhodosporidium*. For example, the nucleic acid can have the sequence SEQ ID NO:1 or SEQ ID NO:2 (both shown below), have a sequence which hybridizes under stringent conditions to SEQ ID NO:1 or 2, or have a sequence which encodes the protein of SEQ ID NO:3.

A vector and transformed host cell containing such a nucleic acid is included within the scope of this invention. By "vector" is meant any nucleic acid molecule or virus containing regulatory elements or reporter genes for the purpose of, but not limited to, expression in prokaryotic or eukaryotic cells or organisms. By "transformed host cell" is meant a host cell into which (or into an ancestor of which) has been introduced, by means of molecular biological techniques, a nucleic acid encoding a DAO from the genus *Rhodosporidium*. After introduction into the cell, this nucleic acid can exist extrachromosomally or become integrated into the host genome.

The present invention also relates to an isolated DAO of the genus *Rhodosporidium*, such as SEQ ID NO:3 (shown below) or a protein which differs from SEQ ID NO:3 by at least one conservative amino acid substitution.

The term "nucleic acid" encompasses both RNA and DNA, including cDNA, genomic DNA, and synthetic (e.g., chemically synthesized or modified) DNA. The nucleic acid may be double-stranded or single-stranded. Where single stranded, the nucleic acid may be a sense strand or an antisense strand. The term "isolated nucleic acid" refers to a nucleic acid which may be flanked by non-natural sequences, such as those of a plasmid or virus. Thus, the nucleic acid can include none, some, or all of the 5' non-coding (e.g., promoter) sequences which are immediately contiguous to the coding sequence. The term, therefore, includes, for example, a recombinant DNA which is incorporated into a vector including an autonomously replicating plasmid or virus, or into the genomic DNA of a prokaryote or eukaryote other than *Rhodosporidium*, or which exists as a separate molecule (e.g., a cDNA or a genomic DNA fragment produced by PCR or restriction endonuclease treatment) independent of other sequences. The term also includes a recombinant DNA or RNA which is part of a hybrid gene encoding an additional polypeptide sequence. Moreover, the term is meant to include nucleic acid fragments which are not naturally occurring as fragments and would not be found in the natural state.

By "hybridizes under stringent conditions" is meant the conditions in which a nucleic acid forms a stable, sequence-specific, non-covalent bond with the nucleic acid of SEQ ID NO:1 or SEQ ID NO:2 in solution or on solid support under the low salt and high temperature conditions regarded as stringent and set forth in Sambrook et al (Molecular Cloning, A Laboratory Manual, Sambrook, J., Fritsch, E. F., and Maniatis, T., 2nd ed. [1989] Cold Spring Harbor Laboratory Press). For example, reference nucleic acids such as SEQ ID NO:1 or SEQ ID NO:2 can be immobilized on nitrocellulose filters, and any other nucleic acids specifically and non-covalently binding to the immobilized reference nucleic acids in the presence of 0.2 X SSC (1.75 g/l NaCl, 0.88 g/l Na₃citrate 2H₂O; pH 7.0) and 0.1% (w/v) sodium dodecylsulfate at 68°C are considered to be hybridized under stringent conditions.

An "isolated D-amino acid oxidase" refers to a DAO which is purified or enriched from its natural environment (e.g., a DAO-rich extract prepared from host cells transformed with a DAO expression vector). A "conservative substitution" refers to a substitution, for example, within one of the following groups: valine, isoleucine, and leucine; aspartic acid and glutamic acid; asparagine and glutamine; serine and threonine; lysine and arginine; and phenylalanine and tyrosine.

The isolation and characterization of the *Rhodosporidium* DAO gene will allow the production of variant forms of the DAO proteins which may have advantageous activities such as a decreased Kₘ for substrate conversion or greater protein stability.

Other features or advantages of the present invention will be apparent from the following drawings and detailed description of several embodiments, and also from the appending claims.

### Detailed Description of the Invention

This invention relates to DAO of the genus *Rhodosporidium*, e.g., *R*. *diobovatum*, *R*. *sphaerocarpum*, *R*. *kratochvilovae*, *R. toruloides*, *R. lusitaniae, R. paludignenum*, *R. dacryoidum*, *R. babjevae*, *and R. fluviale*, and a nucleic acid encoding a *Rhodosporidium* DAO.

Contemplated within the scope of this invention are recombinant nucleic acids or viruses which allow production of DAO protein in a transformed cell or transgenic organism or ease of specific or non-specific mutations within the DAO reading frame. These recombinant nucleic acids or viruses may further include any one of a variety of sequences upstream of the DAO coding sequences, such as strong constitutive promoters; within the DAO coding sequence, such as introns containing *cis*-elements that allow high level expression; or downstream of the DAO coding sequence, such as efficient polyadenylation signals. The invention further includes any cells containing or producing such nucleic acids or viruses, and any DAO proteins produced from such cells.

Without further elaboration, it is believed that one skilled in the art can, based on the above disclosure and the isolation of a DAO protein and a DAO gene from *R*. *toruloides* described below, utilize the present invention to its fullest extent. The following two examples are to be construed as merely illustrative of how one skilled in the art can isolate DAO genes and proteins from any species of the genus *Rhodosporidium*, and are not limitative of the remainder of the disclosure in any way whatsoever.

### Example 1

This example illustrates the purification and characterization of a D-amino acid oxidase active against cephalosporin C from *Rhodosporidium*.

*R*. *toruloides*, obtained from the American Type Culture Collection (No. 10788), was used as a source for the isolation of DAO protein.

The protein was purified as follows: (1) D-alanine was added to cell free extract of *R*. *toruloides* to final concentration of 0.3 M, and mixture heated to 70°C for 4 min. (2) The precipitate was removed by centrifugation at 13,000 x g for 40 min. (3) The supernatant was dialyzed against 1.5 l of buffer A (50 mM potassium phosphate buffer, pH 8.0, 2 mM EDTA, 5 mM β-mercaptoethanol, 1 mM PMSF, and 2.5 g/ml pepstatin A). (4) The dialyzed protein solution was applied to a DEAE-Sephacel column (15 x 2.4 cm) and eluted with buffer A. Fractions with DAO activities were collected and concentrated in an Amicon cell (YM 30). (5) The concentrate was applied to a phenyl-Sepharose CL-4B column (15 x 2.0 cm) that had been equilibrated with 150 ml of buffer B (same as buffer A, but without pepstatin A) supplemented with 20% glycerol. The bound DAO was eluted with buffer B supplemented with 40% glycerol. Fractions with DAO activities were collected and dialyzed against 1.5 l of buffer B containing 10% glycerol. (6) The dialysate was concentrated to 2 ml and chromatographed on a fast protein liquid chromatography (FPLC) Mono Q HR 5/5 column previously equilibrated with 30 ml of buffer B supplemented with 10% glycerol. DAO was eluted immediately after the void volume. Fractions containing enzyme activities were pooled and concentrated to 1 ml. (7) The concentrate was applied to an FPLC gel-filtration Superose 12 HR 10/30 column and eluted with buffer B containing 10% glycerol and 0.25 M NaCl. The active fractions were pooled and stored at -20°C.

The final chromatography used Superose 12 HR column, which allows gel permeation and effectively eliminated minor impurities from the protein sample.

Purified DAO, 0.88 mg, was obtained from about 100 g (wet weight) of *R*. *toruloides* cells with a yield of 21% and a specific activity of 60 U/mg of protein. (DAO unit activity definitions and assay conditions were given in Lee et al., Biotechnol Lett [1994] 16, 467-472.) The preparation yielded a single band in native PAGE. The enzyme was not susceptible to staining with periodic acid/Schiff's reagent, indicating that it was not a glycoprotein.

The molecular weight of the native enzyme was estimated to be 72 kDa by gel-filtration on an FPLC Superose 12 column as compared with a standard curve of reference proteins. SDS/PAGE revealed a single band with a molecular mass of 37 kDa. The results suggest that the native form of *R*. *toruloides* DAO consisted of two subunits of identical size. The NH₂-terminal amino acid sequence of the first 22 residues was determined to be Met His Ser Gln Lys Arg Val Val Val Leu Gly Ser Gly Val Ile Gly Leu Ser Ser Ala Leu Ile (SEQ ID NO:10).

The absorption spectrum of DAO from *R*. *toruloides* showed two maxima, one each at 370 nm and 455 nm, which were typical for flavin chromophores. The E280/E455 ratio, a parameter frequently used to estimate the purity of a flavoprotein, was 8.1. High performance liquid chromatography (HPLC) analyses showed the flavin released from a boiled protein preparation had the same retention time as that of standard flavin adenine dinucleotide (FAD). The concentration of FAD was determined to be 6.34 g/ml, which was extracted from an protein solution of 0.297 mg/ml. Based on this result and SDS/PAGE analysis, a ratio of about 1.73 moles FAD/mole protein was obtained, indicating there was one FAD in each subunit.

The enzyme activity of DAO was measured at various temperatures. The protein exhibited maximal activity at 35°C. The enzyme was active (above 70% of maximal activity) at a temperature range of 30-50°C. The enzyme activity decreased sharply at 60°C to 30% of maximal activity. The enzyme was unstable at temperatures above 35°C. Only 20% and 10% of maximal activity were retained after incubation for 10 min. at 40°C and 45°C, respectively.

The optimal pH for DAO activity was 9.0. The enzyme was active over a pH range of 7.5-10.0. DAO was stable at a pH range of 7.0-8.0 where more than 80% residual activity was detected. However, considerable activity was lost at pH above 8.5 with 70% inactivation at pH 11.0.

The relative activities of the purified DAO on various D-amino acid substrates were measured. The purified enzyme was active on all D-amino acids tested. The best substrate was D-tryptophan, followed by D-methionine, D-phenylalanine, D-alanine, and D-leucine. The enzyme exhibited less activity (<20% of maximal) for D-threonine, D-glutamic acid, D-aspartic acid, and D-lysine. No activity on L-amino acids, including L-alanine, L-proline, L-phenylalanine, and L-methionine, was detected. The apparent Kₘ for each one of D-tryptophan, D-methionine, D-alanine and D-serine was 0.18 mM, 0.22 mM, 0.68 mM, and 3.4 mM, respectively. The enzyme was inhibited by 72%, 49%, and 21% in the presence of p-aminobenzoic acid, benzoic acid, and nicotinic acid, respectively. These aromatic acids were all determined to be competitive inhibitors for *R*. *toruloides* DAO. p-Aminobenzoic acid had the lowest Kᵢ (0.3 mM) among the aromatic acids tested.

The purified DAO protein, having the sequence SEQ ID NO:3 (shown below), was used to convert Ceph C. The enzyme was 95% active on Ceph C as compared with its activity against D-alanine. The apparent Kₘ of the enzyme for Ceph C is 0.65 mM. The yield was 91%, and further incubation did not increase the yield. No exogenous H₂O₂ was required for the reaction.

### Example 2

This example illustrates the isolation and expression of the D-amino acid oxidase gene from *Rhodosporidium*.

The DAO protein from *R*. *toruloides* was purified as described in example 1 above. The last two column chromatographies, Mono Q HR 5/5 and Supersoe 10 HR, were substituted by sodium dodecylsulfate (SDS)/polyacrylamide gel electrophoresis (PAGE). Approximately 300 picomoles of DAO was purified from an SDS/PAGE gel and peptide sequences were determined by the Protein and Nucleic Acid Facility, Stanford University, USA. Three internal peptide sequences (YCQYLARELQ [SEQ ID NO:11], IAGGIDDQAAEPIR [SEQ ID NO:12], and RCTMDSSDP [SEQ ID NO:13]) were determined. To isolate the DAO gene from *R*. *toruloides*, four fully degenerate oligonucleotide primers were synthesized on the basis of the peptide sequences. They were primer 1 5'-AARTAYTGYCARTAYC-3' (SEQ ID NO:6), primer 2 5'-ATNGAYGAYCAYGCNGC-3' (SEQ ID NO:7), primer 3 5' -GCNGCYTGRTCRTCNAT-3 (SEQ ID NO:8), and primer 4 5'-ATGGAYAGYAGYGAYCC-3' (SEQ ID NO:9), where N represents G, A, T or C; Y represents T or C; and R represents A or G.

PCR reactions with chromosomal DNA of *R*. *toruloides* as a template generated no product when primer 1 was used alone. There were several non-specific products synthesized in PCR reactions containing only primer 2, 3, or 4. PCR reactions with primers 2 and 4 also gave several non-specific products. A typical result using primer 1 and 3 was a specific PCR product about 210 bp in length, in addition to non-specific products. When this fragment was used as template, it could be reamplified by primers 1 and 3 in a PCR reaction.

The nucleotide sequence of the fragment was determined. It consisted of the 214 nucleotides shown below: (Lower case letters represent intron sequences, based on the cDNA described below.)
YCQYLARELQ (SEQ ID NO:11) and IAGIDDQA (SEQ ID NO:14), portions of the deduced amino acid sequence encoded by the 214 bp fragment, are identical to a part of the determined DAO protein sequence. The result indicated that the 214 bp DNA fragment is a portion of the gene encoding *R*. *toruloides* DAO. The fragment was then used as a probe for isolating the DAO gene.

Southern blot hybridization with the digoxigenin-labeled 214 bp DNA fragment described above identified a hybridizing fragment of about 3.3 kb. A mini-genomic DNA library was constructed by inserting HindIII-digested genomic DNA fragments with sizes ranging from 3.0 to 3.5 kb into pBluescript SK+. The library was screened with the same probe. Fifteen positive clones with two levels of intensity in signal on autoradiograms were obtained from screening approximately 200 bacterial colonies. Three random clones from each type were selected and analyzed for restriction enzyme profiles of the inserts. The results showed that these clones contained two different DNA inserts, resulting in the two levels of intensity in signal. One type of clones was chosen for further study based on their higher intensity in signal and the hybridization profile of the insert with the 214 bp probe. These clones were found to contain a genomic DNA fragment which contained the DAO gene sequence. The genomic sequence of the DAO gene coding region is shown below: (The lower case letters represent intron sequences, based on the cDNA described below.)

5' and 3' rapid amplification of cDNA ends (RACE) were performed to isolate the cDNA ends for the DAO gene. RACE was performed using RACE kit from Gibco BRL according to manufacturer's protocols. DNA fragments of about 550 bp and 720 bp were amplified in 5'RACE and 3'RACE, respectively. These fragments were cloned into a pGEM-T vector for the determination of nucleotide sequences. The results showed that the two cDNA fragments have a 124-bp overlapping region. The combination of the nucleotide sequences of the two fragments generated an open reading frame (ORF) predicted to encode an amino acid sequence which contains regions with perfect identity to the three internal peptides described above. The coding region of the cDNA of DAO is shown below as. The ORF is 1104 nucleotides long, and encodes a protein of 368 amino acids with a molecular weight of 40,079 Da. The sequence of the protein, from the N-terminus to the C-terminus, is shown below:

Comparison of the nucleotide sequences between the cDNA and the genomic DNA revealed that the DAO gene contains six exons and five introns (see SEQ ID NO:1 above). The sizes of the introns of the DAO gene were relatively small, ranging from 56 bp to 109 bp. Both ends of all five introns contained the exon-intron junction consensus sequence GT......AG.

The nucleotide sequence immediately 5' to the start codon and around the putative translation start site of the DAO gene is in accordance with the consensus sequence CCACCATGGC (SEQ ID NO:16) (Kozak, Nuc Acids Res [1984] 12, 857-872), in which the nucleotide in position -3 (where the A residue of the start codon is designated +1) is always a G or an A. The sequence 5' to the start codon is given below: Consistent with fungal genes, no apparent TATA box is found in the 5' flanking region of the *R*. *toruloides* DAO gene.

In the sequence immediately 3' to the ORF, there is no consensus sequence for the polyadenylation signal. However, the sequence TGTATTGC (SEQ ID NO : 18) located 11 to 18 residues upstream from the poly(A) addition site resembled the sequence YGTGTTYY (SEQ ID NO:17) (where Y represents pyrimidines) known to be involved in the formation of a correct 3' termini in mammalian mRNA. The sequence 3' to the ORF is shown below:

An expression plasmid pDAO-23, carrying the cDNA for *R*. *toruloides* DAO, was constructed using a pET23a vector and introduced into *E. coli* BL21 [DE3]. Isopropyl-1-thio-β-D-galactoside (IPTG)-induced transformants exhibited DAO activity in cell lysates, indicating that active DAO was expressed in *E*. *coli*. SDS/PAGE analyses of the cell lysates revealed a distinct 37 kDa protein band, the estimated size of the DAO subunit, which was not present in the lysate similarly prepared from the pET23a transformed host. The protein was fully induced within 30 min. of IPTG induction. DAO was purified to near homogeneity using Ni-column chromatography. Approximately 4 mg of the protein could be obtained in one step from 250 ml of *E*. *coli* culture. The purified protein showed a specific activity of 1,211 U/mg, a 7-fold increase over that of crude extract and a 20-fold increase over that of the purified *R*. *toruloides* DAO protein. The activity is similar to that of the enzyme prepared by dialysis in the presence of FAD, indicating that the recombinant DAO may contain FAD as a prosthetic group.

A DAO-expressing *E*. *coli* strain, pDAO23-transformed DH5α, was also prepared and has been deposited with the American Type Culture Collection as No. 98485.

### Other Embodiments

It is to be understood that while the invention has been described in conjunction with the detailed description thereof, the foregoing description is intended to illustrate and not limit the scope of the invention, which is defined by the scope of the appended claims.

For example, the DAO proteins of the invention include, but are not limited to, recombinant proteins, natural proteins, and synthetic proteins as well as proteins which are preproteins or proproteins. Recombinant DAO proteins include a DAO with contiguous or non-contiguous amino acid deletions or a DAO containing a signal peptide which allows for transport into the various compartments of a cell, such as the periplasmic space, endoplasmic reticulum, mitochondia, or the extracellular space.

## Claims

1. An isolated nucleic acid comprising the nucleotide sequence of SEQ ID NO:1.

2. A vector containing a nucleic acid of claim 1.

3. A transformed host cell containing a vector of claim 2.

4. An isolated nucleic acid consisting of the nucleotide sequence of SEQ ID NO:1.

5. A vector containing a nucleic acid of claim 4.

6. A transformed host cell containing a vector of claim 5.

## Patentansprüche

1. Isolierte Nukleinsäure, die die Nukleotidsequenz nach SEQ ID Nr. 1 umfaßt.

2. Vektor, der einer Nukleinsäure nach Anspruch 1 umfaßt.

3. Transformierte Wirtszelle, die einen Vektor nach Anspruch 2 umfaßt.

4. Isolierte Nukleinsäure, die aus der Nukleotidsequenz nach SEQ ID Nr. 1 besteht.

5. Vektor, der eine Nukleinsäure nach Anspruch 4 umfaßt.

6. Transformierte Wirtszelle, die einen Vektor nach Anspruch 5 umfaßt.

## Revendications

1. Acide nucléique isolé comprenant la séquence nucléotidique de SEQ ID NO:1.

2. Vecteur contenant l'acide nucléique de la revendication 1.

3. Cellule hôte transformée contenant un vecteur de la revendication 2.

4. Acide nucléique isolé consistant en la séquence nucléotidique de SEQ ID NO:1.

5. Vecteur contenant un acide nucléique de la revendication 4.

6. Cellule hôte transformée contenant un vecteur de la revendication 5.
